(19) Europäisches Patentamt
European Patent Office
Office européen des brevets

(11) EP 0 490 581 B1

(12) **EUROPEAN PATENT SPECIFICATION**

(45) Date of publication and mention of the grant of the patent:
**22.05.1996 Bulletin 1996/21**

(51) Int. Cl.$^6$: **A61K 7/06**

(21) Application number: **91311343.7**

(22) Date of filing: **05.12.1991**

(54) **Cosmetic composition**

Kosmetisches Mittel

Composition cosmétique

(84) Designated Contracting States:
**AT BE CH DE DK ES FR GB GR IT LI NL SE**

(30) Priority: **07.12.1990 GB 9026664**

(43) Date of publication of application:
**17.06.1992 Bulletin 1992/25**

(73) Proprietors:
- **UNILEVER PLC**
  **London EC4P 4BQ (GB)**
  Designated Contracting States:
  **GB**
- **UNILEVER N.V.**
  **NL-3013 AL Rotterdam (NL)**
  Designated Contracting States:
  **BE CH DE DK ES FR GR IT LI NL SE AT**

(72) Inventors:
- **Kealey, George Terence Evelyn**
  **Cambridge CB1 1DT (GB)**
- **Philpott, Michael Paul**
  **Cambridge CB1 3RY (GB)**
- **Williams, Rebecca**
  **Cambridge CB1 3AJ (GB)**

(74) Representative: **Mulder, Cornelis Willem Reinier, Dr. et al**
**Unilever PLC,**
**Patent Division,**
**Colworth House**
**Sharnbrook, Bedford MK44 1LQ (GB)**

(56) References cited:

| | |
|---|---|
| **EP-A- 0 107 885** | **EP-A- 0 116 439** |
| **EP-A- 0 161 811** | **EP-A- 0 273 202** |
| **DE-A- 1 617 477** | **FR-A- 1 603 765** |
| **FR-A- 2 046 949** | **FR-A- 2 609 393** |

- **Fluka Katalog, Fluka Chemie AG, Industriestrasse 5, CH-9470 Buchs, edited 1990, p. 643**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 490 581 B1

## Description

### FIELD OF THE INVENTION

The invention relates to cosmetic and pharmaceutical compositions for topical application to mammalian skin or hair, containing a hair growth promoter which is capable of increasing or maintaining hair growth, especially terminal hair growth on the human scalp.

### BACKGROUND

#### The Hair Growth Cycle

It should be explained that in most mammals, hair does not grow continuously, but undergoes a cycle of activity involving alternate periods of growth and rest. The hair growth cycle can be divided into three main stages, namely:

(i) the growth phase known as anagen, during which the hair follicle penetrates deep into the dermis with the cells of the bulb dividing rapidly and differentiating to form the hair,

(ii) the transitional stage known as catagen, which is heralded by the cessation of mitosis, and during which the follicle regresses upwards through the dermis and hair growth ceases,

(iii) the resting stage known as telogen, in which the regressed follicle contains a small secondary germ with an underlying ball of tightly packed dermal papilla cells.

The initiation of a new anagen phase is revealed by rapid proliferation in the germ, expansion of the dermal papilla and elaboration of basement membrane components. The hair cycle is then repeated many times until, as a consequence of the onset of male pattern baldness, most of the hair follicles spend an increasing proportion of their time in the telogen stage, and the hairs produced become finer, shorter, and less visible; this is known as terminal to vellus transformation.

### PRIOR ART

#### Alleged Baldness Cures

Although there have been many claims in the scientific literature to the promotion or maintenance of hair growth by the topical application of hair tonics and the like, with the possible exception of minoxidil, none has been shown to be sufficiently free from disadvantageous clinical side effects, whether administered topically, orally or systemically, to warrant commercial exploitation as an ethical pharmaceutical, proprietary medicine, or as a cosmetic product. Possibly, the only means which has met with partial success for growing hair on the bald or balding human head is by transplantation of hair to the bald areas. This is, however, an extremely painful operation and is not always successful. Furthermore, it is immediately apparent to the casual observer that the subject has received a hair transplant and it may take many months or even years before hair regrowth, following this operation, assumes an appearance which resembles that of the original naturally growing hair.

Among the many hair regrowth studies that have been reported in the literature, there is included the work of Bazzano as described in PCT International Publication No. WO 85/04577. This publication describes a composition which is useful for increasing the rates of hair growth on mammalian skin, prolonging the anagen phase of the hair growth cycle and for treating various types of alopecias. The composition in question comprises a pyrimidine carbamate.

It has also been reported in US patent no. 4 139 619 to Chidsey assigned to the Upjohn Company, that a topical composition comprising minoxidil as the free base or acid addition salt thereof, or certain specified related iminopyrimidines, is useful in stimulating the conversion of vellus hair to growth as terminal hair, as well as increasing the rate of growth of terminal hair.

In spite of the apparent stimulation of hair growth or regrowth reported independently by Bazzano and Chidsey, following topical application of minoxidil or related compounds, there is general concern that systemic side-effects can result, particularly following topical application of minoxidil. Thus it is generally recognised in the medical literature that the side effects of orally administered minoxidil are very serious, and include fluid retention, tachycardia, dyspnea, gynecomastia, fatigue, nausea and cardiotoxicity. There is also evidence that certain side effects have been experienced following topical application of minoxidil.

It is also reported by Lion Corp., in JP 61151109 that compositions comprising mono-N-long chained acyl basic amino acid lower alkyl ester salt can, together with higher fatty acid having an odd number of carbon atoms, higher

aliphatic alcohol having an odd number of carbon atoms, or their derivatives, can be used for regenerating and growth increasing effect on hair.

DE-1 617 477 (Fischer) discloses a hair tonic which is said to guarantee hair growth. The tonic includes a variety of amino acids as well as four vitamins.

## BACKGROUND TO THE INVENTION

Our own search for effective compositions that could be applied topically to the human scalp in order to promote hair growth, was influenced by the need to discover molecules which were not only effective but also completely safe in use and free from contra indications which would limit their appeal. Furthermore, we were anxious to identify relatively simple molecules in this respect which were easy to synthesis and inexpensive to deploy in a mass market affordable product which would appeal to a large number of potential consumers.

We have noted that the hair follicle has one of the highest rates of cell division in the body. This imposes considerable demands for energy to sustain rapid cell growth. Until recently, little was known of the preferred sources of energy for the hair follicle or the metabolic pathways by which they were utilized.

However, with the recent discovery of a method for maintaining follicle growth and hair production in vitro, we have been able for the first time to investigate the energy metabolism of hair follicles where we can be reasonably certain that experimental observations and conclusions will reflect the behaviour of follicles in vivo.

In the course of these experiments, we have found that the hair follicle can use, as a source of energy, several different fuels in addition to glucose. These include ketone bodies (e.g. 3-hydroxybutyrate, acetoacetate) fatty acids (e.g. palmitate), and glutamine.

Surprisingly, we have found that these alternative fuels do not simply act as a replacement for glucose in terms of energy production. Even in the presence of glucose, we have observed that significant stimulation of linear hair growth rate can be obtained by supplying small amounts of one or more of them.

## DEFINITION OF THE INVENTION

Accordingly, the invention provides a cosmetic method for inducing, maintaining or increasing hair growth, which comprises: topically applying to the body surface where hair growth is desired a composition comprising:

i. an effective amount of from 0.001 to 99% by weight of a hair growth promoter chosen from: glutamine; 3-hydroxybutyric acid; acetoacetic acid; palmitic acid; cosmetically acceptable salts thereof; and mixtures thereof; and

ii. from 1 to 99.999% by weight of a cosmetically acceptable vehicle;

provided that, if the only one of said hair growth promoters present in the composition is glutamine or its cosmetically acceptable salt, and glutamine is not the sole amino acid present in the composition then it is present in an amount by weight which exceeds that of any other amino acid present.

Where glutamine is not the sole amino acid present it preferably constitutes the majority by weight of the total amino acids in the composition.

The composition comprises glutamine or its cosmetically acceptable salt. At least one of: 3-hydroxybutyric acid; acetoacetic acid; palmitic acid; and cosmetically acceptable salts thereof may then also be present.

## DISCLOSURE OF THE INVENTION

### The hair growth promoter

According to the invention, the composition comprises a hair growth promoter chosen from glutamine, or a cosmetically acceptable salt thereof of formula (1);

$$\begin{array}{l} CONH_2 \\ \vert \\ (CH_2)_2 \\ \vert \\ CHNH_2 \\ \vert \\ COOM \end{array} \qquad (1)$$

where M represents hydrogen or a cation chosen from alkali metal salts, ammonium and alkanolammonium (for example M may be sodium) 3-hydroxybutyric acid; acetoacetic acid; palmitic acid; and salts thereof.

As mentioned above the composition may comprise, as hair growth promoter additional or alternative to glutamine one or more of 3-hydroxybutyrate, acetoacetate, and palmitate. Where present these substances are preferably in the form of their sodium salts.

The total amount of the hair growth promoter present in the composition according to the invention is an amount which is sufficient to induce, maintain or increase hair growth. This amount will depend on the effectiveness of the promoter. In general the hair growth promoter will be present in an amount of from 0.0001 to 99% usually from 0.01 to 20% by weight of the composition. This should produce an adequate dose to the skin, which may be repeated as necessary to provide hair growth benefit.

Preservation of the Composition

The composition according to the invention is preferably preserved in such a manner that it will enjoy an extended shelf life following manufacture and prior to sale and use. Ideally the composition will have an indefinite shelf life.

It is accordingly apparent that the hair growth promoter is likely to be prone to attack by bacteria, moulds and fungi and other microbial influences, particularly at pH values near that of the skin that characterise the preferred composition. The shelf-life of the composition can therefore be unacceptably short due to the biodegradation of the hair growth promoter unless steps are taken to preserve the composition.

In order to be preserved, the composition should preferably be free, or substantially free, from viable microbial contaminants that are capable of resulting in microbial spoilage of the composition, and/or biodegradation of the hair growth promoter prior to topical application of the composition to mammalian skin or hair. It is to be understood, however, that the invention is also concerned with compositions, as herein defined, which may contain viable but dormant microorganisms, such as bacterial spores, provided that the conditions of preservation do not result in substantial proliferation of the microorganisms prior to use of the composition.

Examples of methods that can be employed to achieve preservation of the composition, includes the following:

(i) Sterilisation

The composition according to the invention can be preserved by sterilisation to remove or kill substantially all viable microbial contaminants. This can be achieved for example by irradiation using a lethal dose of gamma rays, by heat sterilisation or by ultrafiltration using techniques that are well established in the pharmaceutical industry.

(ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

(iii) Water activity depressants

The composition according to the invention can also be preserved by the inclusion of a water activity depressant such as glycerol, propylene glycol, sorbitol, sugars and salts, for examples alkali metal halides, sulphates and carboxylates. When employing a water activity depressant, sufficient should be incorporated in the composition according to

the invention to reduce the water activity ($\alpha_w$) from 1 to < 0.9, preferably to < 0.85 and most preferably < 0.8, the lowest of these values being that at which yeasts, moulds and fungi will not proliferate.

pH

The hair growth promoter may be susceptable to hydrolysis, particularly when the pH value of the composition is alkaline. It is accordingly preferred that the composition, when aqueous, should have an acid pH value. The preferred pH value of the composition, when aqueous, is from 2 to <7, ideally from 4 to 6.5.

The Vehicle

The composition according to the invention also comprises a solid, semi-solid or liquid cosmetically and/or physiologically acceptable vehicle, to enable the hair growth promoter to be conveyed to the skin at an appropriate dilution. The nature of the vehicle will depend upon the method chosen for topical administration of the composition. The vehicle can itself be inert or it can possess physiological or pharmaceutical benefits of its own.

The selection of a vehicle for this purpose presents a wide range of possibilities depending on the required product form of the composition. Suitable vehicles can be classified as described hereinafter.

It should be explained that vehicles are substances which can act as diluents, dispersants, or solvents for the hair growth promoter which therefore ensure that they can be applied to and distributed evenly over the hair and/or scalp at an appropriate concentration. The vehicle is preferably one which can aid penetration of the hair growth promoter into the skin to reach the immediate environment of the hair follicle. Compositions according to this invention can include water as a vehicle, and/or at least one cosmetically acceptable vehicle other than water.

Vehicles other than water that can be used in compositions according to the invention can include solids or liquids such as emollients, solvents, humectants, thickeners and powders. Examples of each of these types of vehicles, which can be used singly or as mixtures of one or more vehicles, are as follows:

Emollients, such as stearyl alcohol, glyceryl monoricinoleate, glyceryl monostearate, propane-1,2-diol, butane-1,3-diol, mink oil, cetyl alcohol, ispropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polythylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

Propellants, such as trichlorofluoromethane, dichlorodifluoromethane, dichlorotetrafluoroethane, monochlorodifluoromethane, trichlorotrifluoroethane, propane, butane, isobutane, dimethyl ether, carbon dioxide, nitrous oxide;

Solvents, such as ethyl alcohol, methylene chloride, isopropanol, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The amount of vehicle in the composition, including water if present, should preferably be sufficient to carry at least a portion of a selected hair growth promoter to the skin in an amount which is sufficient effectively to enhance hair growth. The amount of the vehicle can comprise the balance of the composition, particularly where little or no other ingredients are present in the composition. Accordingly, the vehicle or vehicles can comprise from 1 to 99.99%, but preferably constitutes a major proportion of the composition, particularly preferably from 50 to 99.5% and ideally from 90 to 99% by weight of the composition.

Perfume

The composition according to the invention can also optionally comprise a perfume in an amount sufficient to make the composition acceptable to the consumer and pleasant to use. Usually, the perfume will form from 0.01 to 10% by weight of the composition.

Activity Enhancer

The composition according to the invention can also optionally comprise an activity enhancer.

The activity enhancer can be chosen from a wide variety of molecules which can function in different ways to enhance the hair growth effects of the hair growth promoter. Particular classes of activity enhancers include other hair growth stimulants, penetration enhancers and cationic polymers, whose presence can further improve the delivery of the hair growth promoter through the stratum corneum to its site of action in the immediate environment of the hair follicle.

Some activity enhancers can also function as vehicles for the ester.

(a) Other Hair Growth Stimulants

i. Examples of other substances which themselves possess the ability to stimulate or increase hair growth include, for example:

Benzalkonium chloride
Benzethonium chloride
Phenol
Estradiol
Diphenhydramine hydrochloride
Chlorpheniramine maleate
Chlorophyllin derivatives
Cholesterol
Salicylic acid
Cystine
Red pepper tincture
Benzyl nicotinate
dl-Menthol
Peppermint oil
Calcium pantothenate
Panthenol
Castor oil
Hinokitiol
Prednisolone
Resorcinol

Further substances which themselves possess the ability to increase the rate of terminal hair growth include:

ii. -1,4 esterified disaccharides described by Choay S.A. in EP-A-O 064 012, having the structure (2):

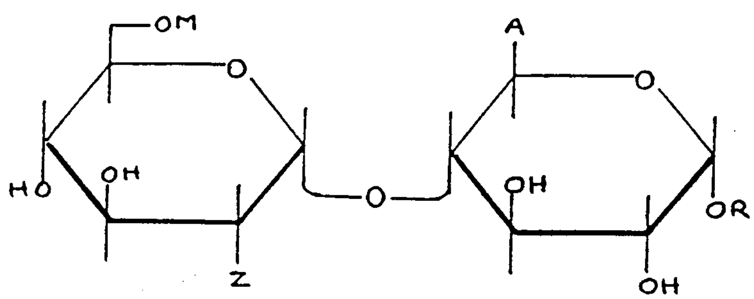

(2)

where

Z represents a functional nitrogen group, such as an azide or a group having the structure -NHB, in which B represents -H or a functional group such as acetyl or sulphate as a salt with an organic or mineral cation;

M represents -H or $SO_3M_1$, where $M_1$ is an organic or metallic cation, particularly an alkali metal; or an acetyl group;

R represents a $C_1$ to $C_4$ alkyl radical, especially methyl; or an aryl radical;

A represents a functional group such as an acid or $-COOR_1$, where $R_1$ represents -H or a $C_1$ to $C_4$ alkyl radical, especially methyl; or a metal, especially an alkali metal;

esterified oligosaccharides as described by Unilever in EP-A-O 211 610, including at least one esterified disaccharide unit consisting of a uronic acid residue having the structure (3):

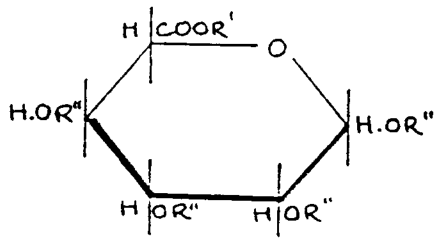

(3)

and a hexosamine residue having the structure (4):

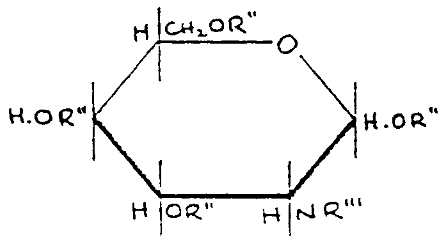

(4)

where

R′ is -H, $C_3$ to $C_{10}$ alkyl or

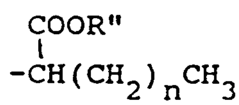

R" is -H, $C_1$ to $C_4$ alkyl, $-CO(CH_2)_mCH_3$, $-SO_3M$,
R‴ is -H, $-CO(CH_2)_mCH_3$, or $-SO_3M$,
M is -H, or a metallic or organic cation
n is 0 or an integer of from 1 to 7, and
m is 0 or the integer 1 or 2;

the groups designated R" being the same or different, one R″ group from each pyranose ring structure being linked by a glycosidic linkage having the configuration α-1,3, α-1,4, β-1,3 or β-1,4; and the -COOR′, $-CH_2OR''$ and -OR″ groups being of either configuration with respect to the pyranose rings;

iii. Minoxidil glucuronides, as described by Unilever in EP-O 242 967,

iv. Minoxidil sulphates, as described by The Upjohn Co. in WO 86/04231, and

v. Minoxidil, and other derivatives thereof as described by The Upjohn Co, in US patent 4 139 619.

Particularly preferred mixtures of minoxidil and a hair growth promoter according to the invention include the following:

Minoxidil and glutathione
Minoxidil and asparagine, and
Minoxidil and glutamine
Minoxidil and 3-hydroxybutyrate (sodium salt)
Minoxidil and acetoacetate (sodium salt)
Minoxidil and sodium palmitate.

vi. Ethylenediaminetetraacetic acid or salts thereof, as described by Redken Laboratories, Inc. in US 4 814 351.

vii. Direct proteoglycanase inhibitors, such as 1,10-phenanthroline, as described by Unilever in EP-0 277 428.

viii.Glycosaminoglycanase inhibitors, as described by Unilever in EP-0 277 428, such as aldonolactones and esterified aldonolactones having the structure (16):

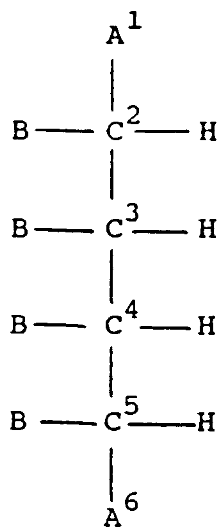

(16)

where

$A^1$ and $A^2$ are -H, $-CH_3$,

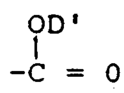

or

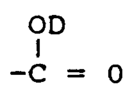

B is OD″ or a lactone linkage to position 1 or 6, or $-NHCOCH_3$

and where

D is -H or $C_2$ to $C_8$ alkyl,

D′ is the remainder of the molecule joined through another C atom at positions 2 to 5 to form a lactone,

D″ is -H or $C_2$ (ie acetyl) to $C_4$ acyl of either configuration with respect to the backbone of this molecule;

preferred examples of which include:

L-Galactono-1,4-lactone
L-Arabino-1,5-lactone
D-Fucono-1,5-lactone
D-Glucaro-1,4-lactone
D-Glucurono-6,3-lactone
Galactaric acid lactone
2-Acetamido-2-deoxygluconolactone
2-Acetamido-2-deoxygalactono-lactone
D-Glucaro-1,4:6,3-dilactone
L-Idaro-1,4-lactone
2,3,5-Tri-0-acetyl-D-glucaro-1,4-lactone
2,5-Di-0-acetyl-D-glucaro-1,4:6,3-dilactone.

ix. Glycosaminoglycanase inhibitors, as described by Unilever in EP 0 277 428, such as monosaccharides and esterified monosaccharides having the structure (17):

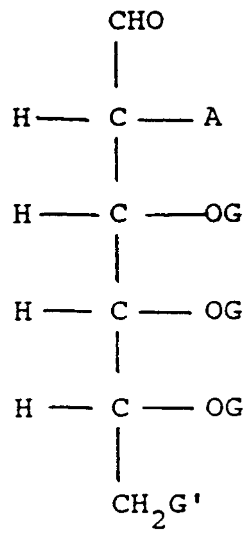

(17)

where
A is -OG or $-NHCOCH_3$
G is -H, $-SO_3M''$, $C_2$ (ie acetyl) to $C_4$ acyl
G′ is -H or -OG
M″ is -H or a metal cation

wherein the functional groups can be in either configuration with respect to the backbone of the above molecule; preferred examples of which include:

N-Acetylglucosamine
N-Acetylgalactosamine
D-Galactosamine
D-Glucosamine-3-sulphate
N-Acetylmannosamine.

x. Glycosaminoglycan chain cellular uptake inhibitors, as described by Unilever in EP 0 277 428, such as hexuronic acid and esters thereof which may be represented by the generic structure (18):

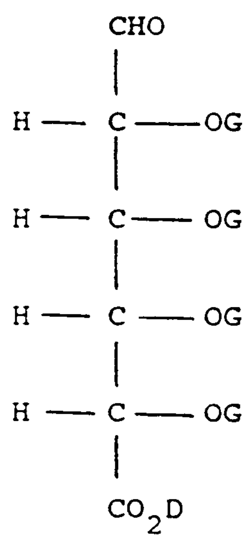

(18)

where
G is -H, $-SO_3M''$, $C_2$ (ie acetyl) to $C_4$ acyl;

D is -H or $C_2$ to $C_8$ alkyl

M″ is -H or a metal cation;

wherein the functional groups can be in either configuration with respect to the backbone of the above molecule;

xi. Chemical inhibitors of glycosidase activity, as described by Unilever in EP 0 334 586, chosen from lactams having the structure (19):

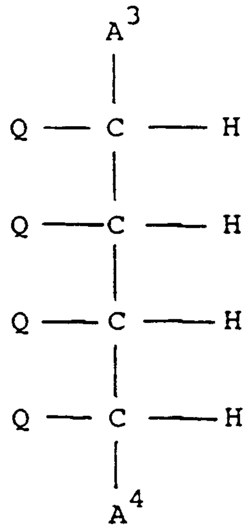

(19)

where $A^3$ and $A^4$ are -H, $-CH_3$,

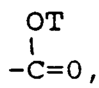

$-CH_2OT$ or

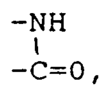

$A^3$ and $A^4$ being the same or different, and at least one of which being the group:

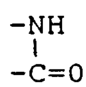

in a lactam ring;

and where Q is -OT′, -NHT′ or a lactam linkage to $A^3$ or $A^4$;

the Q groups being the same or different, and at least one of which is involved in a lactam linkage; and where T is the same or different and is chosen from

-H, $-C_pH_{2p+1}$ or a metal ion,

T′ is -H or $-COC_pH_{2p+1}$, and

p is an integer of from 1 to 22;

provided that:

where any of the Q groups is

-OT′ or -NHT′,

then that group or groups can be of either stereochemical configuration with respect to the plane of the ring, preferred examples of which include:

D-glucaro-1,5-lactam,
L-Galactono-1,4-lactam,
L-Arabino-1,5-lactam,
D-Fucono-1,5-lactam,
D-Glucaro-1,4-lactam,
D-Glucurono-6,3-lactam,
1,2,5-tri-O-acetyl-D-glucurono-6,3-lactam,
2-Acetamido-2-deoxygluconolactam,
2-Acetamido-2-deoxygalactonolactam,
D-Glucaro-1,4:6,3-dilactam,
L-Idaro-1,4-lactam,
2,3,5-Tri-O-acetyl-D-glucaro-1,4-lactam,
2,5-Di-O-acetyl-D-Glucaro-1,4:6,3-dilactam,
D-glucaro-1,5-lactam ethyl ester;

xii. Chemical activators of protein kinase C enzymes, as described by Unilever in EP 0 334 585 chosen from diacylglycerols having the structure (20):

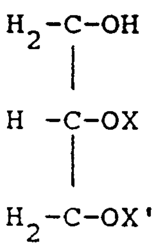

(20)

where X and X' are the same or different and is represented by the grouping:

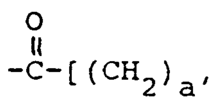

(CH=CH)$_b$] $CH_3$

where x is O or an integer of from 1 to 28, and y is 0 or an integer of from 1 to 5;

the X and X' groups being of either stereochemical configuration with respect to the carbon backbone of the glycerol molecule;

preferred examples of which include:

1,2-Dibutanoyl-rac-glycerol
1,2-Dihexanoyl-sn-glycerol
1,2-Dioctanoyl-rac-glycerol
1,2-Dioctanoyl-sn-glycerol
1,2-Didecanoyl-rac-glycerol
1-Oleoyl-2-acetyl-rac-glycerol
1-Oleoyl-2-acetyl-sn-glycerol
1-Stearoyl-2-arachidonoyl-sn-glycerol
1,2-Distearoyl-rac-glycerol
1,2-Dipentadecanoyl-sn-glycerol
1,2-dipentadecanoyl-rac-glycerol
1,2-Dipalmitoyl-rac-glycerol
1,2-Dipalmitoyl-sn-glycerol

1,2-Diseptadecanoyl-rac-glycerol
1,2-Dioleoyl-sn-glycerol
1,2-Dioleoyl-rac-glycerol
1,2-Diarachidonoyl-sn-glycerol
1,2-Dieicosanoyl-sn-glycerol
1,2-Didoeicosanoyl-rac-glycerol, and
1,2-Dioctaeicosanoyl-sn-glycerol.

xiii.Glycosaminoglycanase inhibitors, as described by Unilever in EP 0 348 184, chosen from aldonomonolactone or alduronomonolactone derivatives having the structure (21):

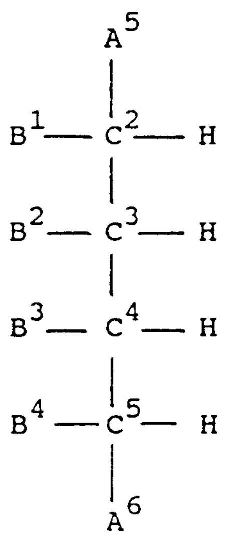

(21)

where
$A^5$ is

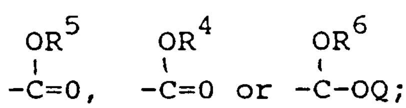

, or ;

$A^6$ is

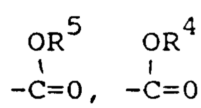

,

or $CH_2OR^6$
$B^1$, $B^2$, $B^3$ and $B^4$ are each chosen from is $OR^5$, $NHR^6$, $NHR^7$ or a lactone linkage to position 1 or 6,and/or an ether linkage to $Q^1$;
said substituents B being the same or different, and being in either configuration, with respect to the backbone of the above structure, on positions $C^2$ to $C^5$ not involved in a lactone ring;
and where
$R^4$ is -H, $C_1$ to $C_{20}$ alkyl, a metal cation, $NH_4$+
or an alkanolamine cation;
$R^5$ is the remainder of the molecule joined through another C atom at positions 2 to 5 to form a lactone;
$R^6$ is -H, $-CH_3$, benzyl or $C_2$ to $C_6$ acyl;

$R^7$ is -H, $-CH_3$, benzyl or $C_3$ to $C_6$ acyl;

$Q^1$ is the remainder of the molecule joined through an ether linkage to either $C^4$ or $C^5$, forming either a pyranose or furanose ring;

provided that, when $A^5$ is

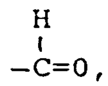

then $A^6$ is

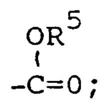

provided also that, when $A^6$ is $CH_2OH$, then one or more of the B substituents is $-CH_3$, $C_2$ to $C_4$ acyl or $NHR^7$;

provided also that, when $A^5$ is

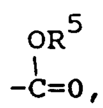

and all $B^1$, $B^2$, $B^3$ and $B^4$ substituents are -OH, then

$A^6$ is

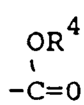

or $CH_2OR^6$, and $R^4$ is $C_1$ or $C_9$ to $C_{20}$ alkyl;

preferred examples of which aldonomonolactone derivatives include:

6-acetyl-galactono-1,4-lactone

6-propionyl-galactono-1,4-lactone

6-butyryl-galactono-1,4-lactone

2-propionamido-2-deoxygluconolactone

2-butyramido-2-deoxygluconolactone

2-propionamido-2-deoxygalactonolactone

2-butyramido-2-deoxygalactonolactone

6-propionyl-2-acetamido-2-deoxygluconolactone diacetyl-6-propionyl-2-acetamido-2-deoxygluconolactone

6-butyryl-2-acetamido-2-deoxygalactonolactone diacetyl-6-butyryl-2-acetamido-2-deoxygalactonolactone

2,3,5,6-tetraacetyl-galactono-1,4-lactone

2,3,5-triacetyl-6-propionylgalactono-1,4-lactone triacetyl-2-propionamido-2-deoxygalactonolactone triacetyl-2-butyramido-2-deoxygluconolactone

6-methyl-glucaro-1,4-lactone

2,3,5,6-tetramethyl-glucaro-1,4-lactone

6-methyl-2,3,5-triacetylglucaro-1,4-lactone

6-methyl-3-methyl-glucaro-1,4-lactone, and

6-methyl-3-acetyl-glucaro-1,4-lactone;

and a preferred example of which alduronomonolactone derivative is:

1,2,5-triacetyl-glucurono-6,3-lactone.

xiv. Glycosaminoglycanase inhibitors, as described by Unilever in EP 0 348 184, chosen from acylated monosaccharides having the structure (22):

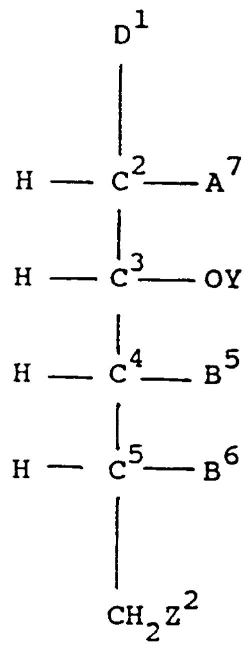

(22)

where

$A^7$ is -OY or $-NHR^8$

$B^5$ and $B^6$ are each chosen from is -OY, or an ether linkage to $D^1$,

$D^1$ is

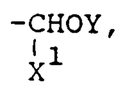

where $X^2$ is an ether linkage either to $C^4$ or $C^5$ forming a pyranose or furanose ring;

Y is -H, $-SO_3M$, $C_2$ to $C_4$ acyl or $C_1$ to $C_{18}$ alkyl;

said substituents $A^7$, $B^5$, $B^6$ and -OY being the same or different, and being in either configuration, with respect to backbone of the above structure;

and where

$Z^1$ is -H or -OY

$R^8$ is -H, $-SO_3M^2$ or $C_3$ or $C_4$ acyl,

$M^2$ is -H, a metal cation, $NH_4+$, or an alkanolamine cation;

provided that, when $R^8$ is -H, then 1 or more of Y is chosen from $-SO_3M^2$ or $C_2$ to $C_4$ acyl;

and mixtures thereof.

Preferred examples of which acylated monosaccharides include:

2-propionamido-2-deoxyglucose

1,3,4,6-tetraacetyl-2-propionamido-2-deoxyglucose

2-butyramido-2-deoxygalactose

1,3,4,6-tetraacetyl-2-butyramido-2-deoxygalactose

2-sulphamido-2-deoxygalactose

2-sulphamido-2-deoxyglucose

2-butyramido-2-deoxymannose

1,3,4,6-tetraacetyl-2-butyramido-2-deoxymannose

2-butyramido-2-deoxyglucose, and

1,3,4,6-tetraacetyl-2-butyramido-2-deoxyglucose.

xv. Esters of pyroglutamic acid, as described by Lever Brothers Company in US patent No. 4 774 255, having the structure (23):

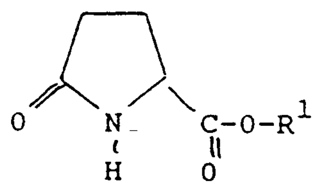

(23)

where

$R^1$ is $C_1$ to $C_{30}$ alkyl, or

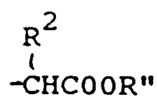

and where $R^2$ and $R^3$ are the same or different and are each represented by H or the grouping (24):

$$[(CH_3)_u, (CH_2OH)_v, (CH_2)_w, (CH_3CH_2)_x, (CHOH)_y, (CH{=}CH)_z]- \quad (24)$$

where

u is zero or 1
v is zero, or the integer 1 or 2,
w is zero, or an integer of from 1 to 21
x is zero, or an integer of from 1 to 4,
y is zero, or the integer 1 or 2,
z is zero, or an integer of from 1 to 4, and
$u + v + w + x + y + z$ is an integer of from 1 to 22;

provided that when the subgrouping (CH=CH) is present, then the total number of carbon atoms in said grouping is from 10 to 22.

Examples of suitable esters of pyroglutamic acid where $R^1$ in structure (23) is $C_1$ to $C_{30}$ alkyl are:

pyroglutamic acid methyl ester
pyroglutamic acid ethyl ester
pyroglutamic acid n-propyl ester
pyroglutamic acid n-butyl ester
pyroglutamic acid n-hexyl ester
pyroglutamic acid n-heptyl ester
pyroglutamic acid n-octyl ester
pyroglutamic acid n-nonyl ester
pyroglutamic acid n-decyl ester
pyroglutamic acid n-undecyl ester
pyroglutamic acid n-dodecyl ester
pyroglutamic acid n-tridecyl ester
pyroglutamic acid n-tetradcyl ester
pyroglutamic acid n-hexadecyl ester
pyroglutamic acid n-octadecyl ester
pyroglutamic acid n-eicosyl ester
pyroglutamic acid iso-propyl ester
pyroglutamic acid 2-methylhexyl ester
pyroglutamic acid 2-ethylhexyl ester
pyroglutamic acid 3,7-dimethyloctyl ester
pyroglutamic acid 2-hexyldecyl ester
pyroglutamic acid 2-octyldodecyl ester
pyroglutamic acid 2,4,4-trimetyl-1-pentane ester

pyroglutamic acid methyloctyl ester

Particularly preferred esters of this group are those where $R^1$ in structure (23) is $C_1$ to $C_{14}$ alkyl, (linear or branched), especially $C_1$ to $C_6$ (linear or branched).

Further examples of preferred esters of pyroglutamic acid, where $R^1$ in structure (23) is

$$-\overset{R^2}{\overset{|}{C}}HCOOR^3,$$

are those where $R^2$ and/or $R^3$ having the structure shown for grouping (24), include straight and branched chain, saturated or unsaturated aliphatic groups having from 1 to 22 carbon atoms, such as the alkyl groups:

methyl
ethyl
propyl
iso-propyl
butyl
iso-butyl
n-valeryl
iso-valeryl
n-caproyl
n-heptyl
n-caprylyl
n-capryl
lauryl
myristyl
palmityl
stearyl, and
arachidyl.

and the $C_{10-22}$ alkenyl groups:

linoleyl
linolenyl
γ-linolenyl
arachidonyl, and
columbinyl.

Further examples of the grouping (24) also include hydroxyalkyl groups having from 1 to 22 carbon atoms, such as:

hydroxymethyl
2-hydroxyethyl
2-hydroxy-n-propyl
3-hydroxy-n-propyl
2-hydroxy-n-butyl
3-hydroxy-n-butyl
4-hydroxy-n-butyl
5-hydroxy-n-valeryl
6-hydroxy-n-caproyl
2,3-dihydroxy-n-propyl
2,3-dihydroxy-n-butyl
12-hydroxystearyl.

Further specific examples of esters of pyroglutamic acid which are particularly suited for use as other hair growth stimulants are:

2-[pyroglutamoyloxy]-propionic acid
methyl-2-[pyroglutamoyloxy]-acetate
ethyl-2-[pyroglutamoyloxy]-n-propionate
ethyl-2-[pyroglutamoyloxy]-n-butyrate
ethyl-2-[pyroglutamoyloxy]-iso-butyrate
ethyl-2-[pyroglutamoyloxy]-n-valerate
ethyl-2-[pyroglutamoyloxy]-n-caproate

ethyl-2-[pyroglutamoyloxy]-n-heptylate
ethyl-2-[pyroglutamoyloxy]-n-caprylate
ethyl-2-[pyroglutamoyloxy]-n-pelargonate
ethyl-2-[pyroglutamoyloxy]-3-hydroxybutyrate
iso-propyl-2-[pyroglutamoyloxy]-n-propionate
iso-propyl-2-[pyroglutamoyloxy]-n-caprylate
n-propyl-2-[pyroglutamoyloxy]-n-propionate
n-propyl-2-[pyroglutamoyloxy]-n-caprylate
stearyl-2-[pyroglutamoyloxy]-n-propionate
12-hydroxystearyl-2-[pyroglutamoyloxy]-n-propionate
stearyl-2-[pyroglutamoyloxy]-n-stearate
palmityl-2-[pyroglutamoyloxy]-n-propionate
linoleyl-2-[pyroglutamoyloxy]-n-propionate
linoleyl-2-[pyroglutamoyloxy]-n-caprylate
lauryl-2-[pyroglutamoyloxy]-n-caprylate
stearyl-2-[pyroglutamoyloxy]-n-caprylate
glyceryl mono(2-[pyroglutamoyloxy]-n-propionate)
glyceryl mono(2-[pyroglutamoyloxy]-n-caprylate), and
glyceryl di(2-[pyroglutamoyloxy]-n-propionate).

xvi. hexosaccharic acids or an acylated hexosaccharic acids, or salts or esters thereof, having the structure (25):

$$\left[\begin{array}{l} COO \\ CHOY^1 \\ CHOY^2 \\ CHOY^3 \\ CHOY^4 \\ COO \end{array}\right]_l \quad X^1_n X^2_m \qquad (25)$$

where

| | |
|---|---|
| $X^1$ | is chosen from H, alkalimetal, ammonium and substituted ammonium counterions; |
| $X^2$ | is chosen from an alkyl or hydroxyaltiyl group having from 1 to 18 carbon atoms; |
| $Y^1$, $Y^2$, $Y^3$ and $Y^4$ | are each chosen from H, an alkyl group having from 1 to 12 carbon atoms, and an acyl group having from 1 to 18 carbon atoms; |
| l | is an integer of from 1 to 3; |
| m and n | are each 0 or the integer 1 or 2; and |
| m+n | is 1 or 2. |

Examples of hexosaccharic acids, in which $X^1$, $Y^1$, $Y^2$, $Y^3$ and $Y^4$ in the above structure are -H, n is 2, and m is 0, include:

Allosaccharic acid
Altrosaccharic acid
Glucosaccharic acid
Mannosaccharic acid
Gulosaccharic acid
Idosaccharic acid
Galactosaccharic acid, and
Talosaccharic acid.

Examples where $X^1$ is a cation, are the monovalent alkali metal cations $Na^+$ and $K^+$.

Further examples where $X^1$ is a cation are substituted ammonium cations, such as diethanolammonium and triethanolammonium cations.

Examples where $X^2$ is an alkyl group are methyl, ethyl, n-propyl, n-butyl, n-octyl and lauryl.

Examples where $Y^1$, $Y^2$, $Y^3$ and $Y^4$ are alkyl groups, are methyl and ethyl.

Examples where $Y^1$, $Y^2$, $Y^3$ and $Y^4$ are acyl group, are acetyl and propionyl.

A particularly preferred hexosaccharic acid is glucosaccharic acid (also known as saccharic acid or glucaric acid, and hereinafter referred to as glucaric acid) having the structure (26):

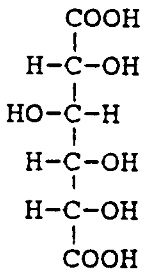

(26)

A particularly stable salt of glucaric acid which is preferred, is the disodium salt.

xvii.aryl-substituted ethylenes having the structure (27)

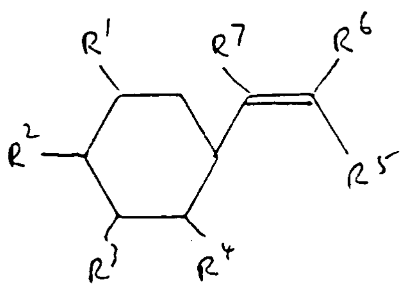

(27)

where $R^1$, $R^2$, $R^3$, & $R^4$ are the same or different, and are chosen from -H, -OH, $-C_nH_{2n+1}$, $-NO_2$, -Cl, -Br, -F and

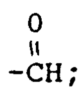

and where $R^5$ & $R^6$ are the same or different, and are chosen from:
-H, -CN,

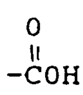, 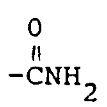 and 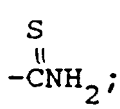

and where $R^7$ is chosen from -H and -OH and where n is an integer of from 1 to 8.

The composition according to the invention can also comprise mixtures of said inhibitors.

Examples of the aryl-substituted ethylenes include

1-carboxy-2-(4-hydroxyphenyl)ethylene 1,1-dicarboxy-2-(4-hydroxyphenyl)ethylene 1,1-dicyano-2-(4-hydroxyphenyl)ethylene 1-carboxy-2-(3,4-dihydroxyphenyl)ethylene 1,1-dicyano-2-(3-hydroxyphenyl)ethylene 1-cyano-1-carboxy-2-(2,5-dihydroxyphenyl)ethylene 1-carboxy-1-cyano-2-(3,4-dihydroxphenyl)ethylene 1,1-dicyano-2-(3,4-dihydroxyphenyl)ethylene 1,1-dicyano-2-(3-methoxy-4,5-dihydroxyphenyl)ethylene 1,1-dicyano-2-(3,4,5-trihydroxyphenyl)ethylene 1-amido-1-cyano-2-(3,4-dihydroxyphenyl)ethylene 1-thioamido-1-cyano-2-(3,4-dihydroxyphenyl)ethylene 1-cyano-2-(4-hydroxyphenyl)ethylene 1,1-dicyano-2-(3-hydroxy-4-nitrophenyl)ethylene 1,1-dicyano-2-hydroxy-2-(4-hydroxyphenyl)ethylene 1,1-dicyano-2-(3-methoxy-4-hydroxyphenyl)ethylene 1,1-dicyano-2-(3,5-dihydroxyphenyl)ethylene 1,1-dicyano-2-hydroxy-2-(3,4,5-trihydroxyphenyl)ethylene
1-carboxy-1-cyano-2-(4-methoxyphenyl)ethylene
1-carboxy-1-cyano-2-(4-fluorophenyl)ethylene
1-carboxy-1-cyano-2-(3-methoxy-4-hydroxyphenyl)ethylene
1-carboxy-1-cyano-2-(3,5-dimethoxy-4-hydroxyphenyl)ethylene
1-carboxy-1-cyano-2-(4-hydroxyphenyl)ethylene
1-carboxy-1-cyano-2-(4-phenylcarboxyaldehyde)ethylene
1-cyano-1-carboxy-2-(2,5-dihydroxyphenyl)ethylene

(b) Penetration Enhancers

As has been stated earlier, the presence of a penetration enhancer can potentiate the benefit of the hair growth promoter by improving its delivery through the stratum corneum to its site of action in the immediate environment of the hair follicle close to the dermal papilla.

The penetration enhancer can accordingly function in a variety of ways. It can for example, improve the distribution of the hair growth promoter on the skin surface or, it can increase its partition into the skin from the composition when applied topically, so aiding its passage to its site of action. Other mechanisms enhancing the benefit of the hair growth promoter may also be involved.

Examples of penetration enhancers include:
2-methyl propan-2-ol
Propan-2-ol
Ethyl-2-hydroxypropanoate
Hexan-2,5-diol
POE(2) ethyl ether
Di(2-hydroxypropyl) ether
Pentan-2,4-diol
Acetone
POE(2) methyl ether
2-hydroxypropionic acid
2-hydroxyoctanoic acid
Propan-1-ol
1,4 Dioxane
Tetrahydrofuran
Butan-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Debenzyl sebacate
Dibutyl phthalate
Dibutyl azelate

Ethyl myristate
Dimethyl azelate
Butyl myristate
Dibutyl succinate
Didecyl phthalate
Decyl oleate
Ethyl caproate
Ethyl salicylate
Isopropyl palmitate
Ethyl laurate
2-ethyl-hexyl pelargonate
Isopropyl isostearate
Butyl laurate
Benzyl benzoate
Butyl benzoate
Hexyl laurate
Ethyl caprate
Ethyl caprylate
Butyl stearate
Benzyl salicylate
2-hydroxypropanoic acid
2-hyroxyoctanoic acid,

Further examples of penetration enhancers include:-

Dimethyl sulphoxide
N,N-Dimethyl acetamide
N,N-Dimethyl formamide
2-Pyrrolidone
1-Methyl-2-pyrrolidone
5-Methyl-2-pyrrolidone
1,5-Dimethyl-2-pyrrolidone
1-Ethyl-2-pyrrolidone
Phosphine oxides
Sugar esters
Tetrahydrofurfural alcohol
Urea
Diethyl-m-toluamide, and
1-Dodecylazacyloheptan-2-one

Further examples of penetration enhancers include surface active agents, preferred examples of which include:

(i) Anionic surface active agents, such as metallic or alkanolamine salts of fatty acids for example sodium laurate and triethanolamine oleate;

alkyl benzene sulphonates, for example triethanolamine dodecyl benzene sulphonate;
alkyl sulphates, for example sodium lauryl sulphate;
alkyl ether sulphates, for example sodium lauryl ether sulphate [2 to 8 EO];
sulphosuccinates, for example sodium dioctyl sulphosuccinate;
monoglyceride sulphates, for example sodium glyceryl monostearate monosulphate;
isethionates, for example sodium isethionate;
methyl taurides, for example Igepon T;
acylsarcosinates, for example sodium myristyl sarcosinate;
acyl peptides, for example Maypons and Lamepons;
acyl lactylates,
polyalkoxylated ether glycollates, for example trideceth-7 carboxylic acid;
phosphates, for example sodium dilauryl phosphate.

(ii) Cationic surface active agents, such as amine salts, for example sapamin hydrochloride;

quartenary ammonium salts, for example Quaternium 5, Quaternium 31 and Quaternium 18;

(iii) Amphoteric suface active agents, such as imidazol compounds, for example Miranol;
N-alkyl amino acids, such as sodium cocaminopropionate and asparagine derivatives;
betaines, for example cocoamidopropylbetaine

(iv) Nonionic surface active agents, such as fatty acid alkanolamides, for example oleic ethanolamide;
esters of polyalcohols, for example Span;
polyglycerol esters, for example that esterified with $C_{12-18}$ fatty acids and one or several OH groups;
polyalkoxylated derivatives, for example polyoxy:polyoxyethylene stearate, and octylphenoxy polyethoxyethanol (TRITON X-100);
ethers, for example polyoxyethylene lauryl ether;
ester ethers, for example Tween;
amine oxides, for example coconut and dodecyl dimethyl amine oxides.

Mixtures of two or more of the above surface active agents can be employed in the composition according to the invention.

(c) cationic polymers chosen from:

Guar Hydroxypropyltrimonium chloride
Quaternium-19
Quaternium-23
Quaternium-40
Quaternium-57
Poly(dipropyldiallylammonium chloride)
Poly(methyl-γ-propaniodiallylammonium chloride)
Poly(diallylpiperidinium chloride)
Poly(vinyl pyridinium chloride)
Quaternised poly (vinyl alcohol)
Quaternised poly (dimethylaminoethylmethacrylate); and mixtures thereof

The amount of activity enhancer, when employed in accordance with the invention, will normally be from 0.1 to 50%, preferably from 0.5 to 25% and most preferably from 0.5 to 10% by weight of the composition.

Other hair growth promoter adjuncts

The composition according to the invention can also contain adjuncts other than those already mentioned, depending on the form of the intended product. It is, for example, possible to include antiseptics, preservatives, antioxidants, emulsifiers and colouring agents, pearlescers, foam boosters, conditioning agents (such as cationic surfactants, cationic polymers, and silicones) and agents such as PFPE (perfluoropolyethylene) for improving hair gloss, which can improve the stability and consumer appeal of the composition.

The composition according to the invention can also be employed as a vehicle for a wide variety of cosmetically or pharmaceutically active ingredients, particularly ingredients which have some beneficial effect other than the promotion of hair growth when applied to the skin.

Process

The invention also provides a process for the preparation of a composition suitable for topical application to mammalian skin or hair which comprises mixing a hair growth promoter as herein defined, with a suitable vehicle to provide a composition according to the invention, in which the hair growth promoter forms from 0.0001 to 99% by weight of the composition.

Product Form and Container

The compositions of the invention can be formulated as liquids, for example as a lotion, shampoo, milk or cream for use in conjunction with an applicator such as a roll-ball applicator, or a spray device such as an aerosol can containing propellant, or a container fitted with a pump to dispense the liquid product. Alternatively, the compositions of the invention can be solid or semi-solid, for example sticks, creams or gels, for use in conjunction with a suitable applicator or simply a tube, bottle or lidded jar, or as a liquid-impregnated fabric, such as a tissue wipe.

The invention accordingly also provides a closed container containing a composition as herein defined.

Surfactants

The composition for use in the method according to the invention can be formulated as a shampoo and will then accordingly comprise one or more surfactants which are cosmetically acceptable and suitable for topical application to the hair. Examples of suitable shampoo surfactants are now given.

Anionic surfactant

The composition of the invention comprises an anionic surfactant which is preferably chosen from alkyl sulphate, alkyl ether sulphate, alkyl sulphonate, alkyl aryl sulphonate, olefin sulphonate, acyl sarcosinate, acyl tauride, acyl isethionate, nonoalkyl sulphosuccinate, dialkylsulphosuccinate, acryl lactylate, acylated $\alpha$-amino acid, alkyl carboxylate, monoalkyl phosphate and dialkyl phosphate.

Suitable examples of anionic surfactants which may be included in the compositions of the invention include:

alkyl sulphates, such as sodium lauryl sulphate [eg. EMPICOL CX available from Albright & Wilson], and triethanolaminde lauryl sulphate [eg. EMPICOL TL40/T, available from Albright & Wilson].

alkylether sulphates, such as sodium lauryl ether sulphate [eg. EMPICOL ESB70, available from Albright & Wilson].

alkyl sulphonates, such as sodium alkane ($C_{13-18}$) sulphonate [eg. HOSTAPUR SAS 30, available from Hoechst].

alkylaryl sulphonates, such as sodium alkyl benzene sulphonate [eg. TEEPOL CM44, available from Shell].

olefin sulphonates, such as sodium olefin sulphonate ($C_{5-18}$) [eg. HOSTAPUR OS, available from Hoechst].

acyl sarcosinates, having the structure: (51)

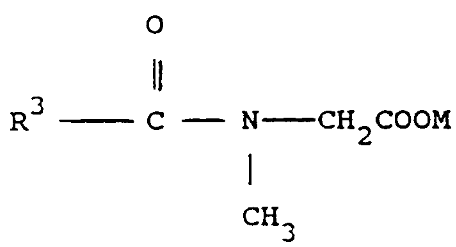

(51)

where

$R^3$ is chosen from $C_{6-14}$ alkyl, and

M is a counterion chosen from alkali metals, ammonium and substituted ammonium such as alkanolammonium.

An example of an acyl sarcosinate having the structure (51), is sodium lauryl sarcosinate [eg. HAMPOSYL L-95, available from Grace].

acyl taurides, having the structure (52):

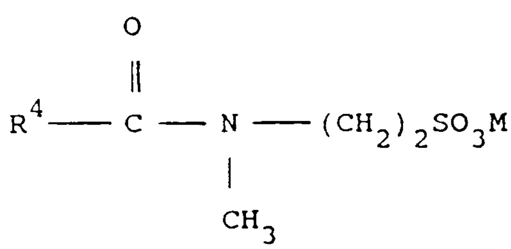

(52)

where $R^4$ is chosen from $C_{8-18}$ alkyl

An example of an acyl tauride having the structure (52) is coconut methyl taurine [eg. FENOPEN TC 42, available from GAF].

acyl isethionates, having the structure (53):

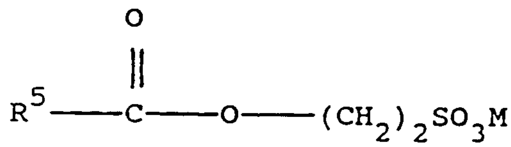

(53)

where $R^5$ is chosen from $C_{8-18}$ alkyl.

An example of an acyl isethionate having the structure (53) is sodium acyl isethionate [eg. JORDAPON C1, available from Jordon].

monoalkyl sulphosuccinates, having the structure (54):

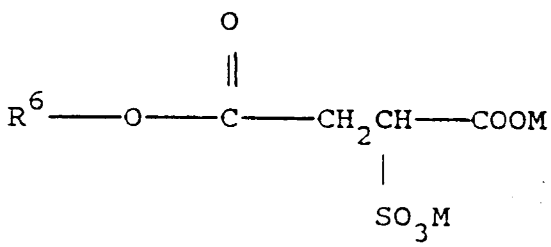

(54)

where

$R^6$ is chosen from $C_{10-20}$ alkyl.

Examples of monoaltiyl sulphosuccinates having the structure (54) include:

sodium lauryl sulphosuccinate [eg EMPICOL SLL, available from Albright & Wilson]
magnesium alkyl sulphosuccinate [eg. ELFANOL 616 Mg, available from AKZO]
sodium lauryl ethoxysulphosuccinate [eg. EMPICOL SDD, available from Albright & Wilson]
coconut monoethanolamide ethoxysulphosuccinate [eg. EMPICOL SGG]
disodium lauryl polyglycolether sulphosuccinate [eg. SURTAGENE S30, available from CHEM-Y]
polyethyleneglycol sulphosuccinate [eg. REWOPOL SBFA 30, available from REWO].

dialkyl sulphosuccinates, having the structure (55):

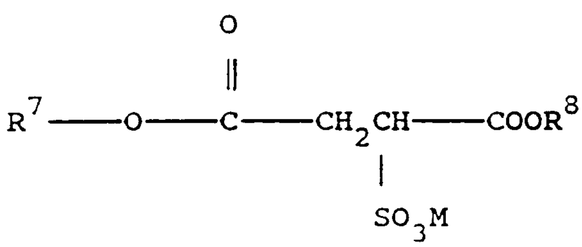

(55)

where $R^7$ and $R^8$ are the same or different, and are chosen from $C_{6-14}$ alkyl.

An example of a dialkyl sulphosuccinate having the structure (55) is sodium dilauryl sulphosuccinate [eg. EMCOL 4500, available from Witco].

acyl lactylates, having the structure (56):

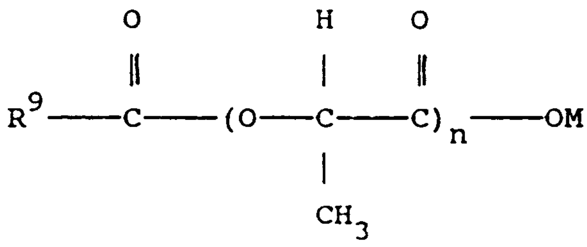 (56)

where
R⁹ is chosen from $C_{6-16}$ alkyl,
and n is 1 or 2.

An example of an acyl lactylate having the structure (6) is decanoyl lactylate [eg. PATIONIC 122A, available from Patterson, CJ].

acylated α-amino acids, such as sodium lauroyl glutamate [eg. ACYLGLUTAMATE LS-11, available from Ajinomoto Co. Inc]

ethyl carboxylates, such as alkyl $C_{12-14}O(EO)_4OCH_2CO_2Na$[eg. AKYPO RLM 38, available from Akzo].

monoalkyl phosphates and dialkyl phosphates, such as dioctyl phosphate.

Amphoteric surfactant

The shampoo compositions of the invention also comprise amphoteric surfactant. Suitable amphoteric surfactants are derivatives of aliphatic quaternary ammonium, phosphonium and sulphonium compounds, wherein the aliphatic radicals contain from 8 to 18 carbon atoms, and may be straight chain or branched, and further contain an anionic water-solubilising group, such as carboxyl, sulphonate, sulphate, phosphate or phosphonate.

Preferred amphoteric surfactants include -

Alkyl betaines, having the structure (57):

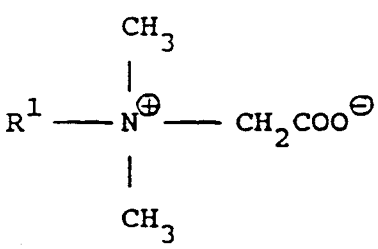 (57)

where
$R^1$ is $C_{1-16}$ alkyl.

An example of an alkyl betaine having the structure (7) is lauryldimethyl betaine (eg. EMPIGEN BB, availabie from Albright & Wilson).

Alkylamidopropyl betaines, having the structure (58):

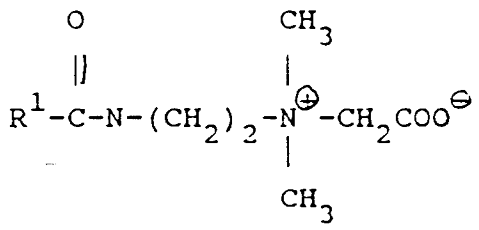

(58)

An example of an alkylamidopropyl betaine having the structure (58) is cocamidopropyl betaine (eg. TEGOBETAIN L7, available from Goldschmidt).

<u>Alkylamphoglycinates or Alkylamphopropionates</u> having the structure (59):

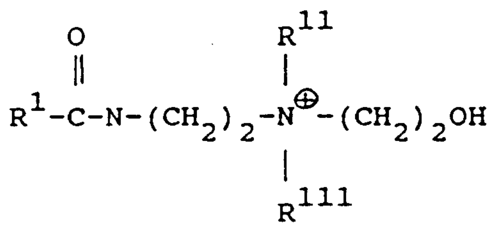

(59)

$R^{11}$ is chosen from H, $CH_2COO^{\ominus}$ and $(CH_2)_2COO^{\ominus}$, and

$R^{111}$ is chosen from $CH_2COO^{\ominus}$ and $(CH_2)_2COO^{\ominus}$

Suitable examples of compounds (59) are cocoamphoglycinate (available from GAF), and cocoamphopropionate.

<u>Sultaines</u>, having the structure (60):

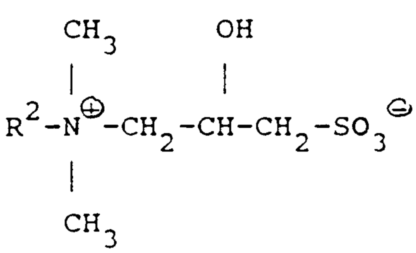

(60)

where

$R^2$ is chosen from $C_{12-16}$ alkyl alkylamido.

An example of a sultaine having the structure (60) is cocamidopropylhydroxysultaine (eg. CYCLOTERIC BET-CS, available from Alcolac).

The most preferred amphoteric surfactant are lauryl dimethyl betaine and cocamidopropyl betaine.

Such amphoteric surfactants can contribute to the foaming of the shampoo of the invention, while ameliorating the harshness of the anionic surfactant.

<u>Nonionic surfactant</u>

The shampoo composition of the invention also comprises alkoxylated or glycosidic nonionic surfactant has an HLB of 8 or more. Above this value nonionics generally form clear isotropic solutions in combination with the other surfactants in the ranges defined above. Preferred nonionic surfactants are polyoxyethylene alkyl esters and polyoxyethylene alkyl ethers and alkyl polyglycosides.

A suitable example of a polyoxyethylene alkyl ester is that having the CTFA designation Polysorbate 80 which is a mixture of oleate esters of sorbitol and sorbitol anhydrides, condensed with approximately 20 moles of ethylene oxide. Also suitable is Polysorbate 20 which is a mixture of laurate esters of sorbitol and sorbitol anhydrides condensed with approximately 20 moles of ethylene oxide.

Polysorbate 80 and Polysorbate 20 are available commercially as TWEEN 80 and TWEEN 20 respectively, from ICI Americas.

Also suitable for use in the compositions of the invention is the polyethylene glycol ether of $C_{9-11}$ alcohol with an average of 8 ethoxy units, which is available commercially as NONIDET LE-8T or as SYNPERONIC 91-8T, and the polyethylene glycol ether of $C_{12-15}$ alcohol with an average of 9 ethoxy units which is available commercially as DOBANOL 25-9.

Particularly useful alkyl polyglycosides include the glycosides of glucose or glucose oligomers where the alkyl chain can be $C_{8-16}$ and the average number of glucose units is 1 to 2. A suitable example is ORAMIX NS 10 which is the glucoside of $C_{10-12}$ fatty alcohol with an average of about 1.5 glucose units.

The amount of surfactant that can be present in the composition according to the invention is from 1 to 20% by weight of the composition.

Use of the hair growth promoter for Inducing, Maintaining or Increasing Hair Growth

The invention also provides for the use of hair growth promoter as herein defined, for topical application to mammalian skin or hair for inducing, maintaining or increasing hair growth.

The compositions according to the invention are primarily intended for topical application to the scalp of the human subject, particularly where the head is already bald or balding, in order to promote the regrowth of terminal hair. The compositions can also be applied prophylactically to the hair and hence the scalp to reduce or prevent the onset of baldness.

The amount of the composition and the frequency of application to the hair and/or scalp can vary widely, depending on personal needs, but it is suggested as an example that topical application of from 0.1 to 5g daily containing from 0.00001 to 1g of a selected chemical inhibitor over the period of at least six months will in most cases result in an improvement in hair growth.

EVALUATION OF EFFICACY OF THE HAIR GROWTH PROMOTERS USING THE IN VITRO HAIR FOLLICLE GROWTH TEST

The effect of compounds on hair growth was assessed using an in vitro test which measures the elongation of isolated human hair follicles in a culture medium.

Isolation of the hair follicle from skin

This test includes the important step of isolating hair follicles having an undamaged hair bulb from skin, preferably from human skin, by microdissection.

The critical step of separating the hair follicle with intact undamaged hair bulb from the subcutaneous fatty tissue in which it is situated accordingly involves severing the hair shaft of the follicle at a point below the epidermis of skin surface, so as to leave the hair bulb intact and undamaged while still bearing a portion of the hair shaft.

Preferably, the hair shaft of the follicle is severed at the dermal-subcutaneous fat interface.

Any suitable cutting instrument can be employed to sever the hair shaft in this manner, but a keratotome or a scalpel are preferred.

The hair bulb with a hair shaft stump attached is then isolated from the skin by mechanically separating the hair from loosely adhering subcutaneous fat which normally surrounds the hair bulb. This is achieved after the dermis or upper layer of the skin has been separated and removed, to avoid damaging the hair bulb as it is pulled away.

The hair bulb together with hair shaft stump attached, is then transferred in an otherwise undamaged and fully functioning, viable state to a nutrient medium.

Culture of the isolated hair follicle

The hair follicles isolated by the technique described herein are transferred to a suitable culture medium for subsequent testing of substances that can then influence their future development.

The procedure now to be described represents a preferred method of culture and testing of hair growth.

In accordance with the preferred method of culture, isolated hair follicles are maintained in 500μl of Williams E medium, either with or without a test hair growth substance, at 37°C in an atmosphere of 5% $CO_2$ + 95% air in individual

wells of a 24 multiwell dish (Corning), which permits detailed measurements to be made of the length of individual hair follicles.

Williams E medium is available from FLOW Laboratory under Catalogue No. 12-502. The formula of Williams E medium is described by Williams GM, et al., in Experimental Cell Research 69 (1971) on page 106.

Hair shaft length measurements can be made on these follicles, using a Nikon Diaphot inverted binocular or similar microscope with eye piece measuring graticules, over a 4 day culture period, during which time the follicles will signficiantly increase in length in response to a hair growth stimulant.

Evaluation of Results

The response of an isolated hair follicle to a test substance, can accordingly be assessed by mesuring the increase in length, if any, in the presence of a test substance against a control.

The *in vitro* method described herein was used to assess the effect of three 'fuels' (hair growth promoters) on hair growth.

In each case isolated human hair follicles were grown in Williams E medium supplemented with glucose (50mM).

The results obtained are summarised in the following Table.

| Hair Growth Promoter in Williams E supplemeted with Glucose | Hair follicle growth * after 6 days |
|---|---|
| Glutamine (2mM) | 4.0 |
| Na 3-hydroxybutyrate (1 mM) | 2.8 |
| Na palmitate (0.5mM) | 1.8 |
| Control | 1.0 |

* these results are relative to a control value of 1.0 and in each case are the mean of 5 different follicles taken from 5 different human subjects (25 follicles in total).

The results summarised above indicated the significant increase in hair growth, as compared with the control, that can be achieved with each of the selected fuels (hair growth promoters). Clearly, glutamine has the most effective.

Examples

The invention is illustrated by the following examples.

Example 1

This Example illustrates a lotion according to the invention which is suitable for topical application to the scalp in order to promote hair growth.

The lotion has the following formulation:

| | % w/w |
|---|---|
| glutamine | 1 |
| ethanol | 99 |
| perfume | q.s. |

Example 2

This Example illustrates a hair tonic which is suitable for application to hair or scalp.

The hair tonic has the following formulation:

| | % w/w |
|---|---|
| glutamine | 2 |
| ethanol | 49 |
| water | 49 |
| perfume | q.s. |

Example 3

This Example also illustrates a lotion which is suitable for topical application to the scalp.
The lotion has the following formulation:

| | % w/w |
|---|---|
| glutamine | 3 |
| propan-2-ol | 10 |
| ethanol | 87 |
| perfume | q.s. |

Example 4

This Example also illustrates a hair tonic which is suitable for application to hair or scalp.
The hair tonic has the following formulation:

| | % w/w |
|---|---|
| glutamine | 3 |
| ethanol | 40 |
| water | 57 |
| perfume | q.s. |

Examples 5 to 8

The following formulations represent lotions which can be used topically in the treatment of bald or balding male or female heads.

| | % w/w | | | |
|---|---|---|---|---|
| | 5 | 6 | 7 | 8 |
| Hydroxyethyl cellulose | 0.4 | - | 0.4 | - |
| Absolute ethanol | 25 | 25 | 25 | 25 |
| Propane-1,2-diol | - | - | 38.4 | 38.4 |
| Butane-1,3-diol | 38.4 | 38.8 | - | - |
| Paramethyl benzoate | 0.2 | 0.2 | 0.2 | 0.2 |
| glutamine | 5 | 4 | 3 | 2 |
| N-acetyl proline | 0.6 | 0.6 | 0.6 | 0.6 |
| Perfume | 1 | 1 | 1 | 1 |
| Water | to 100 | 100 | 100 | 100 |

Examples 9 to 12
(no embodiments of the claimed invention)

The following formulations represent creams which can be used in the treatment of baldness.

| | % w/w | | | |
|---|---|---|---|---|
| | 9 | 10 | 11 | 12 |
| Cetyl alcohol polyoxyethylene (10) | 4 | 4 | 4 | 4 |
| Cetyl alcohol | 4 | 4 | 4 | 4 |
| Mineral oil | 4 | 2 | - | - |
| Paraffin wax | - | 2 | 4 | - |
| Partial glyceride of palmitic and stearic acids | - | - | - | 4 |
| 3-hydroxybutyrate | 2 | - | - | - |
| acetoacetate | - | 2 | - | - |
| sodium palmitate | - | - | 2 | - |
| minoxidil | 0.5 | 0.5 | 0.5 | 0.5 |
| Triethanolamine | 0.75 | 0.75 | 0.75 | 0.75 |
| Butane-1,3-diol | 3 | 3 | 3 | 3 |
| Xanthan gum | 0.3 | 0.3 | 0.3 | 0.3 |
| Preservative | 0.4 | 0.4 | 0.4 | 0.4 |
| Perfume | q.s. | q.s. | q.s. | q.s. |
| Water | to 100 | 100 | 100 | 100 |

Example 13

This Example illustrates a water-in-oil high internal phase emulsion containing glutamine.

The emulsion consisted of 10% by volume oily phase and 90% by weight aqueous phase.

The oily phase and the aqueous phase had the following constitution: kee1

| | % w/w |
|---|---|
| Sorbitan monooleate | 20 |
| Quaternium-18 hectorite | 5 |
| Liquid paraffin | 75 |
| Aqueous phase | |
| glutamine | 1 |
| Xanthan gum | 1 |
| Preservative | 0.3 |
| Perfume | q.s. |
| Sodium chloride (1% w/w solution) | to 100 |

The emulsion was prepared by taking 10 parts by volume of the oily phase and to it adding slowly with stirring 90 parts by volume of the aqueous phase.

The high internal phase water-in-oil emulsion so formed can be applied topically to the scalp, to improve hair growth and regrowth.

The following examples 14 to 18 illustrate shampoos for use in washing the hair and scalp, and for promoting hair growth on the scalp.

Example 14

| | % w/w |
|---|---|
| Sodium lauryl ether sulphate (2 EO) [21% AD] | 41.4 |
| Lauryl dimethylamino acetic acid betaine: [30% AD] | 4 |
| Coconut fatty acid diethanolamine | 1.5 |
| Oleyl triethoxy phosphate (BRIPHOS 03D) | 1 |
| Polyglycol-polyamine condensation resin (POLYQUART H) [50% active] | 1.5 |
| Preservative, colouring matter, salt | 0.58 |
| glutamine | 10 |
| Perfume | q.s. |
| Water | to 100 |

Example 15

| | % w/w |
|---|---|
| Sodium lauryl ether sulphate (2 EO) [100% AD] | 12 |
| POLYMER JR400 | 2.5 |
| BRIPHOS 03D | 2.5 |
| glutamine | 15 |
| Magnesium Sulphate | 5 |
| Perfume | q.s. |
| Water | to 100 |

Example 16

This Example also illustrates a lotion which is suitable for topical application to the scalp.
The lotion has the following formulation:

| | % w/w |
|---|---|
| Glutamine | 5 |
| minoxidil | 1 |
| propan-2-ol | 10 |
| ethanol | 84 |

Example 17

This example illustrates a powder composition according to the invention which can be applied topically to the scalp.

| | % w/w |
|---|---|
| Chemically modified starch | 5 |
| Chemically modified cellulose | - |
| Boric acid | 10 |
| Zinc oxide | 5 |
| glutamine | 3 |
| Minoxidil | 5 |
| Perfume | q.s. |
| Chalk | 10 |
| Talc | to 100 |

Example 18

The following example illustrates a lotion according to the invention which can be applied topically to the scalp to prevent hair loss and stimulate hair regrowth.

| | % w/w |
|---|---|
| glutamine | 7 |
| glucaro-1,4-lactone | 2 |
| ethanol | 16 |
| citric acid | 1.05 |
| water | to 100 |
| pH adjusted to 4.2 with sodium hydroxide | |

Example 19

This example illustrates a hair tonics which is suitable for application to the hair and scalp.

The hair tonic had the following formulation:

| | % w/w |
|---|---|
| glutamine | 2 |
| glucaro-1,5-lactam | 3 |
| ethanol | 50 |
| water | 45 |

Example 20

This example illustrates a shampoo which is suitable for topical application to hair in order to cleanse it, at the same time delivering an inhibitor to the scalp to enhance hair growth or regrowth.

The shampoo had the following formulation:

| | % w/w |
|---|---|
| Triethanolamine lauryl sulphate | 16.8 |
| Coconut diethanolamide | 3.0 |
| Hydroxypropylmethylcellulose (1) | 0.25 |
| Corn syrup (80% solids) (2) | 20.5 |
| Dimethylpolysiloxane (3) | 1.0 |
| Cationic cellulose (4) | 0.5 |
| Ethyl alcohol (SDA 40) | 9.0 |
| Vinyl carboxy polymer (5) | 0.75 |
| glutamine | 8 |
| Perfume, colour, preservative | q.s. |
| Water | to 100 |
| Acid or base to pH: | 6.5 |

1 - Methocel E4M (Dow Chemical)
2 - 42 Dextrose equivalent (Staley 1300)
3 - 60,000 centistokes (Viscasil, GEC)
4 - Polymer JR 400
5 - Carbopol 941 (BF Goodrich)

## Claims

**1.** A cosmetic method for inducing, maintaining or increasing hair growth which comprises topically applying to the body surface where hair growth is desired a composition comprising:

i) an effective amount of from 0.001 to 99% by weight of a hair growth promoter selected from glutamine; cosmetically acceptable salts thereof; and mixtures thereof; and

ii) from 1 to 99.999% by weight of a cosmetically acceptable vehicle;

provided that if glutamine or its cosmetically acceptable salt is not the sole amino acid present in composition then it is present in an amount by weight which constitutes the majority by weight of the total amino acids in the composition.

**2.** A method according to claim 1 wherein the glutamine or its cosmetically acceptable salt is accompanied by one or more of 3-hydroxybutyric acid, acetoacetic acid; palmitic acid; and cosmetically acceptable salts thereof.

**3.** A method according to claim 1 or claim 2 in which the hair growth promoter forms from 0.01 to 20% by weight of the composition.

**4.** A method according to any one of claims 1 to 3 in which the composition further comprises an activity enhancer.

**5.** A method according to claim 4 in which the activity enhancer is a hair growth stimulant.

**6.** A method according to claim 5 in which the hair growth stimulant is minoxidil.

**7.** A method according to claim 4 in which the activity enhancer is a penetration enhancer.

**8.** A method according to claim 7 in which the penetration enhancer is a surface active agent.

**9.** A method according to claim 4 in which the activity enhancer is a cationic polymer.

**10.** A method according to any one of the preceding claims in which the composition has a pH value of from 2 to 7.

**11.** A method according to any one of the preceding claims in which the composition is a shampoo or hair conditioner.

**12.** The cosmetic use of a composition comprising:

i) an effective amount of from 0.001 to 99% by weight of a hair growth promoter selected from glutamine; cosmetically acceptable salts thereof; and mixtures thereof; and

ii) from 1 to 99.999% by weight of a cosmetically acceptable vehicle;
provided that if glutamine or its cosmetically acceptable salt is not the sole amino acid present in the composition then it is present in an amount by weight which constitutes the majority by weight of the total amino acids in the composition;
for inducing, maintaining or increasing hair growth by topical application.

**13.** The cosmetic use of glutamine, cosmetically acceptable salts thereof, and mixtures thereof for inducing, maintaining or increasing hair growth by topical application.

**14.** A composition suitable for topical application to mammalian skin or hair for inducing, maintaining or increasing hair growth which comprises:

i) an effective amount of from ≧ 0.001% by weight of a hair growth promoter selected from glutamine; cosmetically acceptable salts thereof; and mixtures thereof;

ii) from ≧ 1% by weight of a cosmetically acceptable vehicle;
provided that if glutamine or its cosmetically acceptable salt is not the sole amino acid present in the composition then it is present in an amount by weight which constitutes the majority by weight of the total amino acids in the composition; and

iii) from 1 to 20% by weight of one or more surfactants selected from anionic, amphoteric and nonionic surfactants, and mixtures thereof.

**Patentansprüche**

**1.** Kosmetisches Verfahren zur Induzierung, Beibehaltung oder Erhöhung eines Haarwachstums durch topisches Applizieren einer Zusammensetzung, die

(i) eine wirksame Menge von 0,001 bis 99 Gew.-% eines unter Glutamin, kosmetisch akzeptablen Salzen hiervon und Gemischen hiervon ausgewählten Haarwachstumsförderers und

(ii) 1 bis 99,999 Gew.-% eines kosmetisch akzeptablen Trägers umfaßt,

wobei gilt, daß, wenn Glutamin oder ein kosmetisch akzeptables Salz hiervon nicht die alleinige in der Zusammensetzung vorhandene Aminosäure ist, Glutamin in einer gewichtsprozentualen Menge vorhanden ist, die den Hauptgewichtsanteil an den gesamten Aminosäuren in der Zusammensetzung ausmacht,
auf die ein Haarwachstum erfordernde Körperoberfläche.

**2.** Verfahren nach Anspruch 1, wobei das Glutamin oder ein kosmetisch akzeptables Salz hiervon zusammen mit einem oder mehreren Bestandteilen aus 3-Hydroxybuttersäure, Acetoessigsäure, Palmitinsäure und kosmetisch akzeptablen Salzen hiervon vorliegt.

**3.** Verfahren nach Anspruch 1 oder Anspruch 2, wobei der Haarwachstumsförderer 0,01 bis 20 Gew.-% der Zusammensetzung bildet.

4. Verfahren nach einem der Ansprüche 1 bis 3, wobei die Zusammensetzung des weiteren einen Aktivitätsverstärker umfaßt.

5. Verfahren nach Anspruch 4, wobei der Aktivitätsverstärker ein Haarwachstumsstimulans ist.

6. Verfahren nach Anspruch 5, wobei das Haarwachstumsstimulans Minoxidil ist.

7. Verfahren nach Anspruch 4, wobei der Aktivitätsverstärker ein Penetrationsverstärker ist.

8. Verfahren nach Anspruch 7, wobei der Penetrationsverstärker ein grenzflächenaktives Mittel ist.

9. Verfahren nach Anspruch 4, wobei der Aktivitätsverstärker ein kationisches Polymer ist.

10. Verfahren nach einem der vorhergehenden Ansprüche, wobei die Zusammensetzung einen pH-Wert von 2 bis 7 aufweist.

11. Verfahren nach einem der vorhergehenden Ansprüche, wobei die Zusammensetzung ein Shampoo oder ein Haarkonditioniermittel ist.

12. Kosmetische Verwendung einer Zusammensetzung, die

(i) eine wirksame Menge von 0,001 bis 99 Gew.-% eines unter Glutamin, kosmetisch akzeptablen Salzen hiervon und Gemischen hiervon ausgewählten Haarwachstumsförderers und
(ii) 1 bis 99,999 Gew.-% eines kosmetisch akzeptablen Trägers umfaßt,

wobei gilt, daß, wenn Glutamin oder ein kosmetisch akzeptables Salz hiervon nicht die alleinige in der Zusammensetzung vorhandene Aminosäure ist, Glutamin in einer gewichtsprozentualen Menge vorhanden ist, die den Hauptgewichtsanteil an den gesamten Aminosäuren in der Zusammensetzung ausmacht,
zur Induzierung, Beibehaltung oder Erhöhung eines Haarwachstums durch topische Applikation.

13. Kosmetische Verwendung von Glutamin, kosmetisch akzeptablen Salzen hiervon und Gemischen hiervon zur Induzierung, Beibehaltung oder Erhöhung eines Haarwachstums durch topische Applikation.

14. Zusammensetzung mit Eignung zur topischen Applikation auf Säugetierhaut oder Säugetierhaar zur Induzierung, Beibehaltung oder Erhöhung eines Haarwachstums, die

i) eine wirksame Menge von ≧0,001 Gew.-% eines unter Glutamin, kosmetisch akzeptablen Salzen hiervon und Gemischen hiervon ausgewählten Haarwachstumsförderers,

ii) ≧1 Gew.-% eines kosmetisch akzeptablen Trägers, wobei gilt, daß, wenn Glutamin oder ein kosmetisch akzeptables Salz hiervon nicht die alleinige in der Zusammensetzung vorhandene Aminosäure ist, Glutamin in einer gewichtsprozentualen Menge vorliegt, die den Hauptgewichtsanteil an den gesamten Aminosäuren in der Zusammensetzung ausmacht, und

iii) 1 bis 20 Gew.-% eines oder mehrerer unter anionischen, amphoteren und nichtionischen grenzflächenaktiven Mitteln und Gemischen hiervon ausgewählter grenzflächenaktiver Mittel umfaßt.

**Revendications**

1. Procédé cosmétique pour induire, maintenir ou augmenter la croissance des cheveux qui consiste à appliquer localement à la surface du corps où la croissance des cheveux est voulue une composition comprenant :

i) une quantité efficace de 0,001 à 99% en poids d'un promoteur de croissance des cheveux choisi parmi la glutamine, ses sels cosmétiquement acceptables et leurs mélanges, et
ii) de 1 à 99,999% en poids d'un véhicule cosmétiquement acceptable ;
à la condition que si la glutamine ou son sel cosmétiquement acceptable n'est pas le seul aminoacide présent dans la composition, alors elle est présente en une quantité en poids qui constitue la majorité en poids des aminoàcides totaux dans la composition.

2. Procédé selon la revendication 1, dans lequel la glutamine ou son sel cosmétiquement acceptable est accompagnée par un (ou plusieurs) acide 3-hydroxybutyrique, acide acétoacétique ; acide palmitique, et leurs sels cosmétiquement acceptables.

3. Procédé selon la revendication 1 ou 2, dans lequel le promoteur de croissance des cheveux forme de 0,01 à 20% en poids de la composition.

4. Procédé selon l'une quelconque des revendications 1 à 3, dans lequel la composition comprend en outre un rehausseur d'activité.

5. Procédé selon la revendication 4, dans lequel le rehausseur d'activité est un stimulant de la croissance des cheveux.

6. Procédé selon la revendication 5, dans lequel le stimulant de la croissance des cheveux est le minoxidil.

7. Procédé selon la revendication 4, dans lequel le rehausseur d'activité est un rehausseur de la pénétration.

8. Procédé selon la revendication 7, dans lequel le rehausseur de la pénétration est un agent tensioactif.

9. Procédé selon la revendication 4, dans lequel le rehausseur d'activité est un polymère cationique.

10. Procédé selon l'une quelconque des revendications précédentes, dans lequel la composition a un pH de 2 à 7.

11. Procédé selon l'une quelconque des revendications précédentes, dans lequel la composition est un shampooing ou un conditionneur capillaire.

12. Utilisation cosmétique d'une composition qui comprend :

i) une quantité efficace de 0,001 à 99% en poids d'un promoteur de croissance des cheveux choisi parmi la glutamine ; ses sels cosmétiquement acceptables ; et leurs mélanges ; et
ii) de 1 à 99,999% en poids d'un véhicule cosmétiquement acceptable ; à la condition que si la glutamine ou son sel cosmétiquement acceptable n'est pas le seul aminoacide présent dans la composition alors elle est présente en une quantité en poids qui constitue la majorité en poids des aminoacides totaux dans la composition;

pour induire, maintenir ou augmenter la croissance des cheveux par application locale.

13. Utilisation cosmétique de glutamine, de ses sels cosmétiquement acceptables et de leurs mélanges pour induire, maintenir ou augmenter la croissance des cheveux par application locale.

14. Composition appropriée pour l'application locale à la peau ou aux cheveux des mammifères pour induire, maintenir ou augmenter la croissance des cheveux qui comprend :

i) une quantité efficace $\geqq$ 0,001 en poids d'un promoteur de croissance des cheveux choisi parmi la glutamine, ses sels cosmétiquement acceptables ; et leurs mélanges ;
ii) $\geqq$ 1% en poids d'un véhicule cosmétiquement acceptable ;

à la condition que si la glutamine ou son sel cosmétiquement acceptable n'est pas le seul aminoacide présent dans la composition, alors elle est présente en une quantité en poids qui constitue la majorité en poids des aminoacides totaux dans la composition ; et

iii) de 1 à 20% en poids d'un (ou plusieurs) tensioactif choisi parmi les tensioactifs anioniques, amphotères et non ioniques et leurs mélanges.